# EUROPEAN PATENT APPLICATION

(11) **EP 4 316 578 A1**
(43) Date of publication of application: **07.02.2024**
(21) Application number: 22781557.8
(22) Date of filing: 29.03.2022
(51) Int. Cl.: A61N 5/06, A61N 1/40, A61N 1/32, A61N 7/00, A61B 5/00, A61N 5/067

(54) **SKIN TREATMENT DEVICE AND CONTROL METHOD THEREFOR**

(30) Priority: 01.04.2021 KR 20210042634
(71) Applicant: Lutronic Corporation, Goyang-si, Gyeonggi-do 10534 (KR)
(72) Inventor: KIM, Jae Song, Goyang-si, Gyeonggi-do 10448 (KR); LE, Viet-Hoan, Goyang-si, Gyeonggi-do 10532 (KR)
(74) Representative: Michalski Hüttermann & Partner Patentanwälte mbB
(86) International application number: PCT/KR2022/004385
(87) International publication number: WO 2022/211438

(57) **Abstract**

The present invention relates to a skin treatment device and a control method therefor, and the present invention provides a skin treatment device and a control method therefor, the device comprising: a treatment unit for performing treatment by moving on the surface of the skin through operation by the user and transmitting energy to a plurality of treatment locations; a sensing unit for sensing location information about the treatment unit while the treatment unit moves; a storage unit for storing, on the basis of the information sensed by the sensing unit, information about the location at which treatment is performed; and a control unit for controlling the operation of the treatment unit on the basis of the information about the location at which treatment is performed.

## Description

### [Technical Field]

The present disclosure relates to a skin treatment device and a control method thereof, and more particularly, to a skin treatment device for performing a treatment by transferring energy to a plurality of locations while moving on a skin surface, and a control method thereof.

### [Background Art]

Recently, a treatment technology, such as transferring energy for a treatment to human tissues to transform or remove a state of human tissues, has been widely applied. Therefore, treatment devices using various types of electromagnetic waves, such as laser beams, flash lamps, RF radio frequency waves, microwaves, and ultrasounds, have been developed.

In particular, skin treatment devices for treating skin lesions having various types of energy sources and treating various lesions using the energy sources have been widely used. Such a device is also disclosed in Korean Patent No. 10-1269970. As an example, a skin treatment device using light performs a treatment such that light having a specific wavelength penetrates inside a skin and is absorbed by various tissues, such as collagen, hair follicles, and hemoglobin located inside the skin according to wavelength characteristics and the absorbed light is converted into heat energy in the tissues to change a state of the tissues.

These skin treatment devices generally include a handpiece, and a user performs a treatment at a plurality of treatment locations, while moving the handpiece on a skin surface. However, when the treatment is performed repeatedly at a specific location, excessive energy may be transmitted to a corresponding tissue to damage the tissue.

### [Disclosure]

### [Technical Problem]

The present disclosure is to provide a skin treatment device capable of preventing tissue damage caused by excessive energy as a user repeatedly performs a treatment on a specific location in treating a skin surface, while moving a handpiece, and a control method thereof.

### [Technical Solution]

In one aspect, a skin treatment device includes: a treatment unit configured to move on a skin surface by a user's motion and perform a treatment by delivering energy to a plurality of treatment locations; a detecting unit configured to detect location information of the treatment unit while the treatment unit is moving; a storage unit configured to store information on a treatment location on which treatment has been performed based on information detected by the detecting unit; and a controller configured to control a treatment operation based on information on the treatment location on which the treatment has been performed.

Here, the storage unit may cumulatively stores information on a plurality of treatment locations on which the treatment has been performed. In addition, the storage unit may cumulatively store information on a plurality of treatment locations on which the treatment has been performed and information on a treatment order of each treatment location.

Specifically, while the treatment is performed by the treatment unit, the storage unit may generate a treatment map recording the treatment location information and cumulatively record the treatment location information on the treatment map. In this case, while a treatment is performed through a first treatment path, the storage unit may record treatment location information corresponding to the first treatment path in a first treatment map, and while a treatment is performed through a second treatment path, the storage unit may record treatment location information corresponding to the second treatment path in a second treatment map.

In addition, the skin treatment device may further include: a display unit configured to display the treatment map to a user while the treatment is performed by the treatment unit.

Also, the detecting unit may detect location information of the treatment unit by detecting horizontal movement information according to reference coordinates and rotation information of the reference coordinates, while the treatment unit is moving. As an example, the detecting unit may include an image sensor configured to acquire movement information by acquiring an image of the skin surface in real time while the treatment unit is moving and a gyro sensor configured to detect rotation information of the treatment unit.

Also, when the treatment unit is located in a previously treated treatment location or within a preset distance from the previously treated treatment location, the controller may control the treatment unit not to transmit energy to the skin surface or controls the treatment to be performed with relatively low output energy. Specifically, the treatment unit may automatically transmit energy to the skin surface according to a preset mode while moving along the skin surface by a user's motion, and when the treatment unit is located in a previously treated treatment location or within a preset distance from the previously treated treatment location, the controller may control the treatment unit not to transmit energy to the skin surface or controls the treatment to be performed with relatively low output energy.

In another aspect, a control method of a skin treatment device includes: detecting location information of a treatment unit by a detecting unit, while moving on a location of a patient's skin surface by a user's motion; operating a treatment unit to transmit energy to the skin surface to perform a treatment; and cumulatively storing information on a treatment location on which a treatment has been performed by the treatment unit based on information detected by the detecting unit, wherein, in the step of performing the treatment, the controller controls the operation of the treatment unit in consideration of location information of the treatment unit and the information on the treatment location on which the treatment has been performed.

In another aspect, a control method of a skin treatment device includes: generating a treatment map for recording information on a treatment location on which a treatment has been performed; comparing a first location on which a treatment is scheduled with information on a previously treated location recorded in the treatment map; when the first location satisfies a preset treatment condition according to a comparison result of the comparing operation, transferring energy to the first location to perform a treatment; and when the treatment is performed on the first location, recording the first location as a previous treatment location on the treatment map.

### [Advantageous Effects]

According to the present disclosure, even if a treatment is performed, while moving a handpiece, an overtreatment may be prevented by considering information on a treatment location at which the treatment has already been performed, and a uniform treatment may be performed without a missing part.

### [Description of Drawings]

FIG. 1 is a view illustrating an optical treatment device according to an embodiment of the present disclosure;
FIG. 2 is a block diagram schematically illustrating main components of FIG. 1;
FIG. 3 is a block diagram illustrating an operational relationship of the main components of FIG. 2;
FIG. 4 is a graph illustrating an example of a method in which a detecting unit of FIG. 2 detects location information of a treatment unit, while a handpiece is moving;
FIG. 5 is a view illustrating an example of a method of storing a previously treated treatment position in a storage unit;
FIG. 6 is a view illustrating an example of a treatment map for explaining control contents of a controller of FIG. 3;
FIG. 7 is a graph illustrating contents of a treatment operation of the controller according to FIG. 6;
FIG. 8 is a flowchart illustrating the main steps of a control method of a skin treatment device of the present embodiment; and
FIG. 9 is a flowchart illustrating a control method of a skin treatment device according to the present embodiment in more detail.

### [Mode for Disclosure]

Hereinafter, a skin treatment device according to an embodiment of the present disclosure will be described with reference to the accompanying drawings. In the following description, a location relationship between constituent elements will be principally described based on drawings. The drawings may be exaggerated, omitted or schematically drawn for the purpose of convenience or clarity. Accordingly, the present disclosure is not limited thereto. Various variations and modifications are possible in the component parts and/or arrangements of the subject combination arrangement within the scope of the disclosure.

Hereinafter, the term 'skin treatment device' includes all treatment devices for treating mammals including humans. These skin treatment devices include treatment devices using various energy sources used for the purpose of improving the condition of a lesion or tissue on a skin surface. In the present embodiment, a description is given using a treatment device using treatment light, such as a laser, as an energy source, but the present disclosure is not limited thereto. For example, the present disclosure may also be applied to various treatment devices that perform a treatment by using light sources other than a laser or by using different energy sources, such as RF, ultrasound, and sound waves.

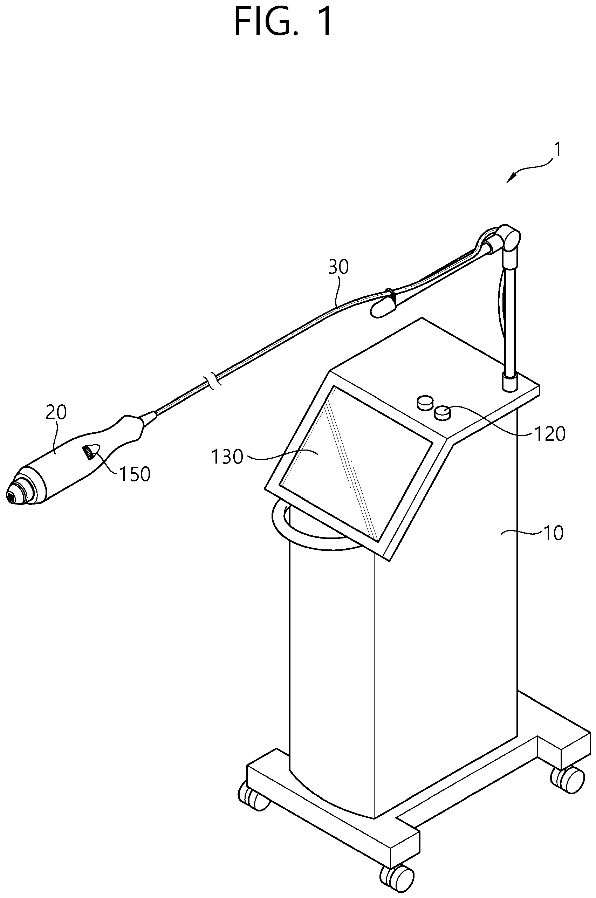

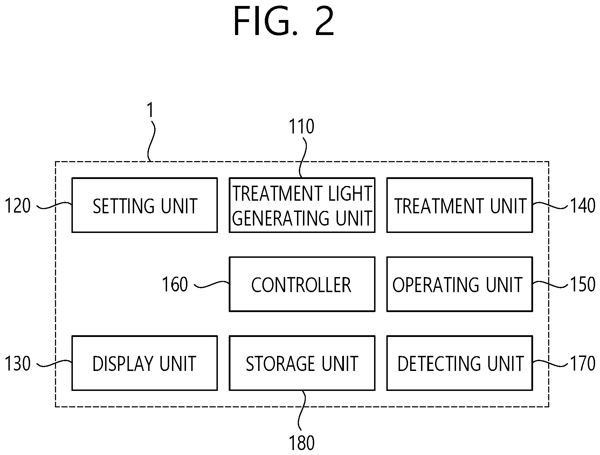

Hereinafter, a skin treatment device according to an embodiment of the present disclosure will be described with reference to FIGS. 1 and 2.

FIG. 1 is a perspective view illustrating a skin treatment device according to an embodiment of the present disclosure. As shown in FIG. 1, a skin treatment device 1 according to the present embodiment includes a main body 10, a handpiece 20 and a connecting portion 30 connecting the main body 10 and the handpiece 20.

The main body 10 forms a main body structure of the skin treatment device, and various components are installed therein. For example, a treatment light generating unit 110 as a source for generating a treatment energy source and various optical elements for transmitting treatment light to the handpiece may be provided inside the main body 10. An outer surface of the main body 10 is provided with a setting unit 120 for operating the treatment device or setting operation contents and a display unit 130 for displaying various information for the user.

The handpiece 20 is a component for performing a skin treatment and is configured for a user to hold by hand. The user performs a treatment at various locations on a skin surface, by changing locations of the handpiece while moving the location of the hand with the handpiece 20 held in the hand.

The handpiece 20 includes a treatment unit 140 that delivers treatment energy to a treatment location. The treatment unit 140 of the present embodiment irradiates treatment light generated by the treatment light generating unit 110 to a treatment location on the skin surface. An outer surface of the handpiece 20 is provided with an operating unit 150 for regulating an operation of the handpiece. In addition, a detecting unit 170 or the like for detecting various types of information during a treatment may be installed in the handpiece.

The connecting portion 30 is configured to connect the main body 10 and the handpiece 20 described above. The connecting portion 30 includes a light transmitting portion that forms an optical path from the treatment light generating unit of the main body to the treatment unit of the handpiece and a signal line that transmits various control signals generated by a controller of the main body or the operating unit of the handpiece.

FIG. 2 is a block diagram schematically illustrating the main components of FIG. 1. Hereinafter, each component of FIG. 1 will be described in more detail with reference to FIG. 2.

As shown in FIG. 2, the treatment light generating unit 110 is provided inside the main body. As described above, the treatment light generating unit 110 is configured to generate treatment light and may be configured using various types of light sources. In the present embodiment, the treatment light generating unit 110 is configured to include a laser medium capable of oscillating a laser and a resonator. However, in addition, it is also possible to configure the treatment light generating unit 110 using various light sources, such as LED, LD, and halogen lamps.

As an example, the treatment light generating unit 110 may use Nd;YAG or Alexandrite as a laser medium. Therefore, treatment light generated by the treatment light generating unit may be a laser having a wavelength (Nd:YAG) in the range of 1060 nm to 1070 nm or a wavelength (Alexandrite) in the range of 750 nm to 760 nm, and may be configured, more specifically, as a layer having a wavelength of approximately 1064 nm or a laser having a wavelength of approximately 755 nm. However, the skin treatment device according to the present embodiment is for rejuvenation of the skin or treatment of pigmented lesions, a wavelength effective for the corresponding treatment is selected, and in addition to this, it is also possible to use a laser medium that generates other wavelengths.

A plurality of optical elements for modulating and transmitting treatment light are disposed on one side of the treatment light generating unit. Treatment light passing through these optical elements is transferred to the treatment unit 140 of the handpiece via the light transmitting portion of the connecting portion 30. The light transmitting portion may include at least one optical fiber or may include a light transmission structure including a plurality of relay lenses.

The treatment unit 140 is a component provided in the handpiece and transmitting energy to the skin surface, which is a treatment region. Here, the treatment unit 140 may refer to the handpiece itself or may refer to a sub-component of the handpiece that contributes to delivering energy to the skin surface. As an example, the treatment unit 140 of the present embodiment includes a plurality of optical elements provided in the handpiece and forms a path along which treatment light travels in the handpiece. Accordingly, treatment light transmitted through the connecting portion may be received and irradiated to a treatment location through an end portion of the handpiece.

Meanwhile, the controller 160 is a component that controls each component of the skin treatment device 1. The controller 160 operates the skin treatment device according to the contents set by the user through the setting unit 120, the contents operated by the user through the operating unit 150 of the handpiece, or the contents stored an internal memory (not shown).

As an example, the controller 160 controls a parameter or an irradiation pattern of treatment light by controlling the treatment light generating unit or an optical element disposed on a travel path of treatment light. For example, an irradiation time, an irradiation frequency, a pulse width, a pulse waveform, an output, a wavelength, etc. of treatment light may be adjusted by controlling an operation of a flash lamp or shutter that excites the laser. Alternatively, a spot size of treatment light may be adjusted by controlling a movable lens forming an optical path. In addition to irradiation of treatment light, the controller 160 may control operation of various components provided in the main body and the handpiece, such as performing a required calculation upon receiving detected information from the detecting unit, determining the contents displayed on the display unit, or controlling an operation of a cooling unit if the cooling unit is provided.

In addition, the skin treatment device 1 according to the present embodiment further includes the detecting unit 170 that detects movement information of the treatment unit and a storage unit 180 that stores information on a treatment location at which a treatment is performed based on the information detected by the detecting unit. Also, the controller 160 is configured to control the treatment operation of the treatment unit 140 based on the information on a treatment location which has already been treated, stored in the storage unit 180.

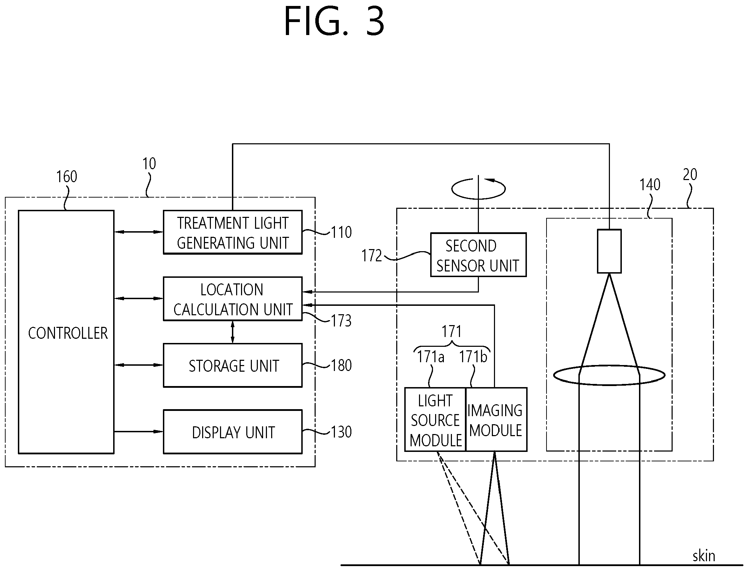

FIG. 3 is a block diagram illustrating an operational relationship of the main components of FIG. 2. Hereinafter, the detecting unit and the storage unit of the present embodiment will be described in more detail with reference to FIG. 3.

As shown in FIG. 3, at least a portion of the detecting unit 170 is provided in the handpiece and detects location information of the handpiece 20, i.e., location information of the treatment unit 140, while the handpiece is moved by the user's moving motion. Therefore, while the handpiece 20 performs a treatment in a plurality of different locations, while moving along the skin surface, the detecting unit 170 may detect location information at the time of treatment to detect locations at which a treatment has been performed.

Here, the detecting unit 170 may detect location information at a preset period or nonperiodically detect location information at a specific point in time. For example, when the treatment unit 140 repeatedly irradiates treatment light at a preset period, the detecting unit 170 may periodically detect location information at the time of irradiation of treatment light at the same period as a treatment light irradiation period. Alternatively, when treatment light is irradiated singly by the user's shot operation, the detecting unit 170 may detect location information at each treatment light irradiation time (or the user's shot operation time).

Specifically, the detecting unit 170 of the present embodiment includes a first sensor unit 171 detecting horizontal movement information of the handpiece, a second sensor unit 172 detecting axial rotation information of the handpiece, and a location calculation unit 173 that calculates location information of the handpiece based on the information detected by the first sensor unit and the second sensor unit.

The first sensor unit 171 is a component that detects location movement information in a horizontal direction on the skin surface. As an example, the first sensor unit 171 may use an optical sensing method. As shown in FIG. 3, the first sensor unit 171 of the present embodiment includes a light source module 171a and an imaging module 171b to acquire an image of the skin surface to which treatment light is irradiated, analyze the acquired image, and detect location information of the handpiece that moves.

Specifically, the light source module 171a irradiates image light to the skin surface, and the imaging module 171b acquires an image by receiving image light reflected from the skin surface. The light source module 171a of the present embodiment irradiates a vertical cavity surface irradiating laser (VCSEL), and accordingly, the imaging module 171b may acquire a skin surface image of a preset area of the skin surface.

Using this, the first sensor unit 171 may obtain an image of the skin surface at a preset period while the handpiece is moving, and analyze the continuously obtained image to monitor the location movement information in real time. For example, movement information may be detected by analyzing and deriving an overlapping portion between two consecutively acquired images or by deriving a change in locations of a feature point (e.g., a point or bump on the skin) that is a reference between two consecutively acquired images. Accordingly, the first sensor unit 171 may detect horizontal location movement information of the handpiece in real time.

Meanwhile, the second sensor unit 172 is configured to detect axial rotation information of the handpiece. Here, the axis refers to an axis formed in a direction perpendicular to the skin surface as a concept opposite to the horizontal direction described above and may refer to a central axis of the handpiece in a longitudinal direction. For reference, the detecting unit (in particular, the first sensor unit) 170 may have a unique reference coordinate system in order to detect and recognize location information of the handpiece. Here, the reference coordinate system is a two-dimensional coordinate system of xoy, which may be a coordinate system fixed to the handpiece structure (e.g., the side on which the operating unit is formed is fixed in a +y direction). Here, it is assumed that the first detecting unit 170 recognizes a movement location of the handpiece as coordinates on the reference coordinate system. At this time, even if the first detecting unit detects that the handpiece moves to the same coordinates (x, y), an actual movement location is inevitably different between a case in which the handpiece rotates to move and an otherwise case. Accordingly, the second sensor unit 172 is configured to detect rotation information of the handpiece, that is, rotation information of the reference coordinate system.

The location calculating unit 173 of the detecting unit 170 is a component of calculating location information of the handpiece upon receiving signals detected by the first sensor unit 171 and the second sensor unit 172. In the present embodiment, the location calculation unit 173 is provided in the main body and is configured to receive detection information from the first and second sensor units through the connecting portion, but, in addition to this, a location calculation unit may also be provided in the handpiece. As described above, the location detecting unit 170 detects location information of the handpiece by combining the horizontal direction movement information of the first sensor unit 171 and the rotation information acquired by the second sensor unit 172. In this case, even though various movements, such as the user holding the handpiece turns and holds the handpiece, rotates the wrist during movement, or rotating the handpiece around the elbow, occur, the detecting unit 170 may detect accurate movement location information of the handpiece 20.

Of course, when location information is recognized based on images, as in the case of the first sensor unit 171, it is also possible to detect minute rotation information between two consecutive images if there are at least two reference feature points. However, if the user quickly turns and holds the handpiece or if a location of the detecting unit or a location to which treatment light is irradiated is eccentric from the center of a cross-section of the handpiece, it is difficult to accurately recognize the rotational motion of the handpiece only with the method as that of the first sensor unit. Therefore, the detecting unit 170 of the present embodiment may further include the second sensor unit 172 detecting rotation information in addition to the first sensor unit 171 to improve location detection accuracy.

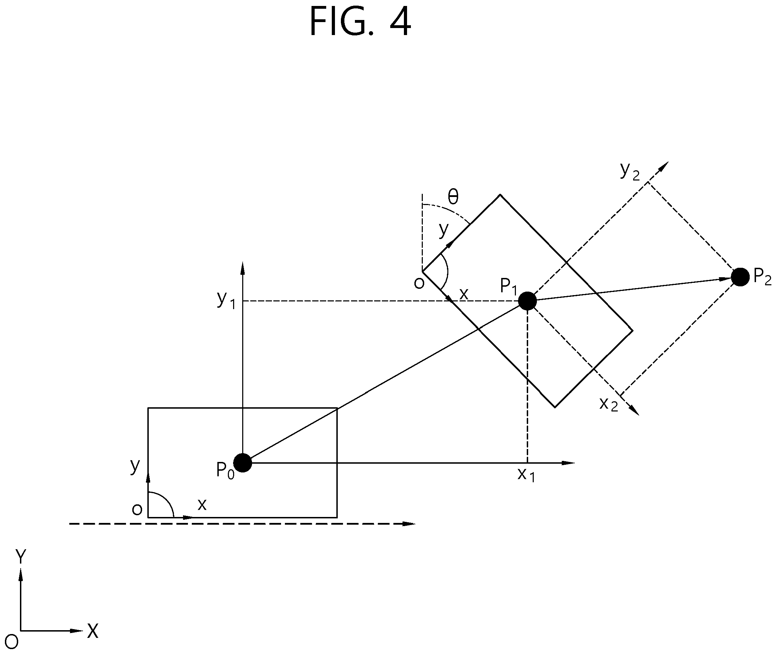

FIG. 4 is a graph illustrating an example of a method in which the detecting unit of FIG. 2 detects location information of the treatment unit while the handpiece is moving. Hereinafter, an example in which the sensor unit detects location information of the handpiece is described with reference to FIG. 4.

In FIG. 4, the OXY coordinate system is a global coordinate system for displaying a location relationship on the skin surface, and the oxy coordinate system is the reference coordinate system of the detecting unit. Also, P0 is a starting location of the handpiece (or a location of the treatment unit or an irradiation location of treatment light), P1 is a first movement location, and P2 is a second movement location.

As shown in FIG. 4, it can be seen that, while moving from P0 to P1, movement is made by (x 1, y1) on the reference coordinate system at P0 and the reference coordinate system is rotated by θ at the location of P1. In addition, it can be seen that, while moving from P1 to P2, movement is made by (x2, y2) based on the reference coordinate system at P1. In this case, a change in location from P0 to P2 based on the global coordinate system may be calculated as (x1+x2cosθ+y2sinθ, y1-x2sinθ+y2cosθ).

Here, the change in the horizontal location at each location is detected by the first sensor unit 171, the change in the rotational location is detected by the second sensor unit 172, and the location calculation unit 173 calculates location information on the global coordinate system based on information acquired by each sensor unit. In addition, for a movement location (e.g., P3, P4, etc.) after P2, it is possible to detect horizontal direction movement information and rotation information and cumulatively calculate based on this to detect each location information on the global coordinate system.

Here, the locations of P0 to P2 may be locations of the handpiece in each measurement period when the detecting unit periodically detects location information during movement of the handpiece. Alternatively, the locations of P0 to P2 may be locations of the handpiece at the time of treatment (in the present embodiment, locations to which treatment light is irradiated).

In the above, an example of calculating location information by the detecting unit has been described with reference to FIG. 4. However, the aforementioned method is described as a simple example for convenience of description, and it is noted that location information may be detected by various calculation methods using horizontal movement information and rotation information.

Referring back to FIG. 3, the skin treatment device of the present embodiment further includes the storage unit 180 storing a treatment location on which a treatment has already been performed. While the user moves the handpiece and performs a treatment on a plurality of treatment locations, it is not easy to visually check the location on which the treatment has already been performed. Meanwhile, since energy is transmitted to the treatment location, if the treatment is performed again on the location where treatment has already been completed, excessive energy may be transmitted and skin damage may occur. To this end, the storage unit 180 of the present embodiment may store previously treated treatment location information, and the controller 160 may perform a treatment in consideration of the previous treatment location information stored in the storage unit, thereby solving the problem.

As shown in FIG. 3, the storage unit 180 receives information on previously treated locations detected by the detecting unit 170 and stores the same. In FIG. 3, the storage unit is shown as a separate component from the location calculation unit 173 of the detecting unit, but the present disclosure is not limited thereto. For convenience of description, components are divided based on functions, and the location calculation unit and the storage unit may be configured as a single component to perform the aforementioned functions.

The storage unit 180 cumulatively stores information on treatment locations which have been already treated, while the handpiece moves to perform a treatments on a plurality of treatment locations . At this time, the storage unit 180 may simultaneously store previously treated treatment location information and information on a treatment order of each location. Furthermore, it is also possible to include not only treatment location information but also treatment parameter information performed at each treatment location.

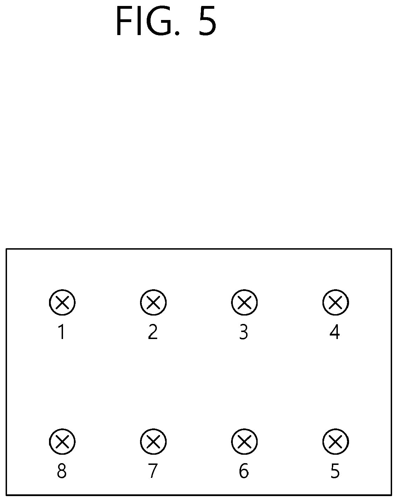

FIG. 5 is a view illustrating an example of a method of storing previously treated treatment locations in a storage unit. As shown in FIG. 5, the storage unit 180 generates a treatment map while treatment is performed by the treatment unit 140 and cumulatively stores treatment location information where the treatment is performed. Such a treatment map may be created based on the aforementioned overall coordinates, and a plurality of treatment locations are cumulatively recorded on the treatment map. Also, the treatment order in each treatment location may also be displayed together (refer to FIG. 5).

While the user is moving the handpiece and performing a treatment on a plurality of locations, the previously treated location is updated and stored in the treatment map in real time. Therefore, prior to performing a treatment on a preset location, it is possible to determine a relative location of a corresponding location with other treatment locations.

When treatment is performed in such a manner that the treatment unit 140 automatically irradiates treatment light according to a preset period, the treatment map may cumulatively record treatment locations through one treatment path, and when a new treatment path starts, a treatment location may be recorded on a new treatment map. Here, one treatment path may refer to a treatment path in which the user performs a treatment in one continuous motion. In another aspect, one treatment path may refer to a procedure in which a treatment is performed by continuously irradiating treatment light at a preset period from the start of the irradiation of treatment light by the user through a start operation to a stop operation. The storage unit 180 cumulatively records treatment locations where a treatment is performed during a first treatment path on a first treatment map while the first treatment path is being performed, and when a second treatment path starts, a treatment location according to the second path may be cumulatively recorded on a separate second treatment map.

As another embodiment, the treatment map generated by the storage unit 180 may be displayed to the user through the display unit 130 described above. The user may then design treatment contents in consideration of the previously treated location information displayed on the treatment map. At this time, the display unit 130 may display the treatment map on a virtual human main body model (for example, a face model for a face treatment) in an overlapping manner, or display the treatment map on a previously captured treatment region image of a patient in an overlapping manner.

Referring back to FIG. 3, the controller 160 controls the treatment operation by operating various components, and in the present embodiment, the controller 160 controls the treatment operation based on the previously treated treatment location information. As described above, the detecting unit 170 detects the location information of the handpiece in real time while the user moves the handpiece, and the storage unit 180 cumulatively stores information on the previously treated treatment location. Therefore, before performing a treatment at a preset location, the controller 160 may detect location information of a corresponding location through the detecting unit 170 and compare the detected location information with previous treatment locations stored in the storage unit 180 to determine a relative location relationship, such as whether the detected location information is a previously treated location or how close the detected location information is to the previously treated location. Then, the controller 160 controls treatment operation contents at the corresponding location in consideration of the location relationship.

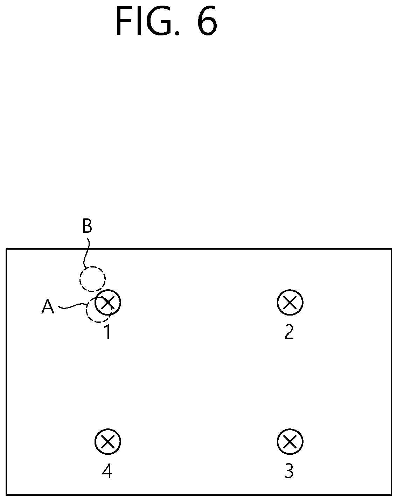

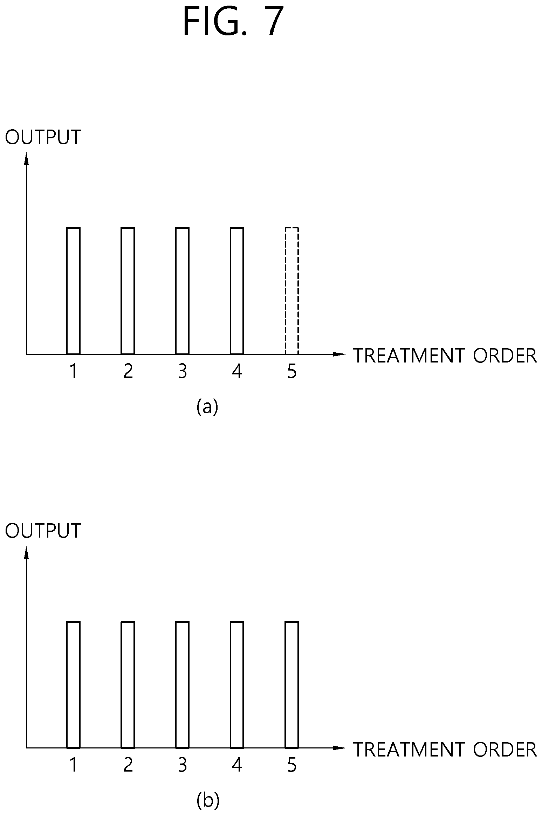

FIG. 6 illustrates an example of a treatment map for explaining control contents of the controller of FIG. 3, and FIG. 7 is a graph illustrating treatment operation contents of the controller according to FIG. 6. Hereinafter, examples of treatment operation control considering a treatment location will be described in detail with reference to FIGS. 6 and 7.

FIG. 6 is a treatment map generated by the storage unit, and the corresponding treatment map displays four treatment locations previously treated in a corresponding treatment path and a treatment order. When the user intends to perform a treatment at a fifth treatment location, if the fifth scheduled treatment location is A and is the same location as a first location (if at least a portion overlaps), the controller 160 takes this into consideration and controls as follows.

a of FIG. 7 shows an output of treatment light according to a first control example. As shown in a of FIG. 7, compared to irradiating treatment light with normal output up to a fourth treatment location, the fifth treatment location is the same location as a previously treated location, so a treatment may be controlled not to be performed but to be skipped. However, (a) of FIG. 7 shows an example in which a treatment is not performed in a previously treated location, but in addition to this, it is also possible to control to perform a treatment by irradiating treatment light with a relatively low output (e.g., 50% of a normal output) an it is also possible to control to perform a treatment by adjusting a magnitude of the output according to an overlap rate of treatment light in two treatment locations (i.e., as the overlap rate increases, treatment light with a relatively low output is irradiated).

b of FIG. 7 shows an output of treatment light according to a second control example. In a of FIG. 7, the treatment contents are controlled by considering the relative locations of the scheduled treatment locations and previously treated locations, but in b of FIG. 7, it is also possible to control treatment contents in consideration of a treatment order in addition to the relative locations. For example, when a time (3T) for which an output of treatment light equal to three times an irradiation period T of treatment light has elapsed, energy is regarded to be dispersed by the previously irradiated treatment light, and the fifth treatment light may be controlled to perform a treatment with a normal output as shown in b of FIG. 7. However, if a scheduled treatment location is the same as the fourth treatment location, it is also possible to control as shown in a of FIG. 7 because energy is not dispersed by the previous treatment.

In this manner, the controller 160 may minimize tissue damage caused by a treatment by controlling treatment contents in a location to be treated based on the previously treated location information. In a and b of FIG. 7, the control example of the treatment contents has been described, but this has been simplified for convenience of description, and various control scenarios may be designed and reflected in the control in consideration of a treatment lesion, a treatment part, treatment intensity, etc.

In addition, in the above, the description is given based on the case in which the location to be treated is the same location as the previously treated location, but the present disclosure is not limited thereto. If the location to be treated is within a preset distance from a previous treatment location as shown in B of FIG. 6, energy may not be transmitted to the corresponding location or energy with a relatively low output may be transmitted like a or b of FIG. 7 as in the case of the same location (for example, the case A).

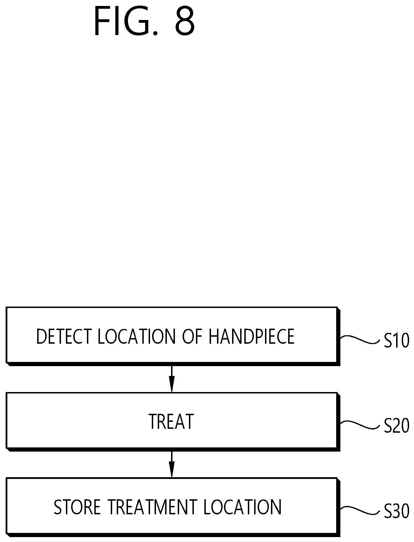

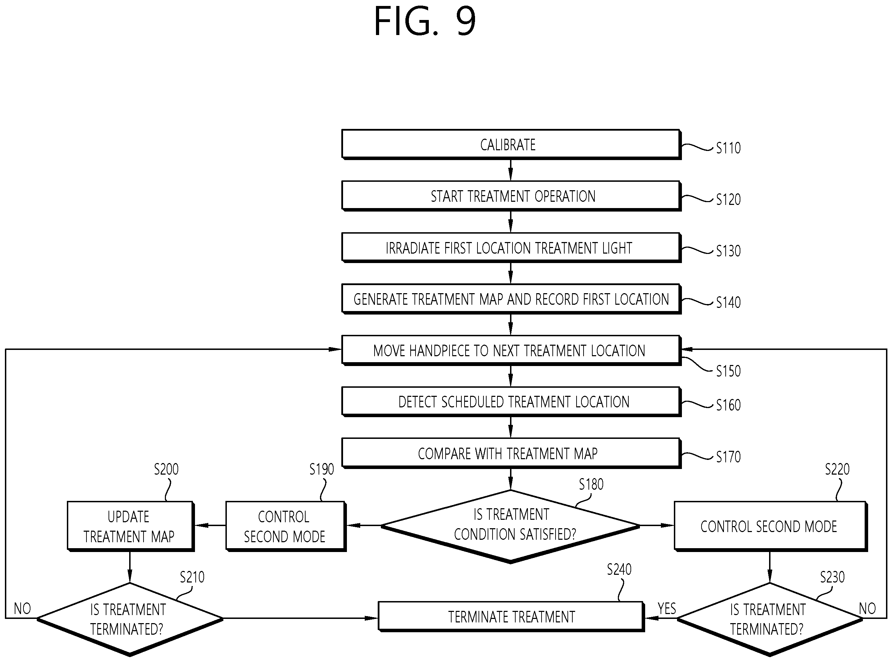

Hereinafter, a control method of the skin treatment device and a treatment method using the same according to the present embodiment will be described with reference to FIGS. 8 and 9.

FIG. 8 is a flowchart illustrating the main steps of a control method of a skin treatment device according to the present embodiment. As shown in FIG. 8, the skin treatment device performs a step of detecting location information of a handpiece while moving on a skin surface by a user's motion (S10).

In the location information detection step, the location information of the handpiece is detected in real time by the detecting unit 170. In this step, as described above, it is possible to calculate a real-time location of the handpiece by detecting horizontal movement information according to the reference coordinate system detected by the first sensor unit 171 and rotation information of the reference coordinate system detected by the second sensor unit 172.

Also, the handpiece performs a step of performing a treatment by irradiating treatment light to the skin surface using the treatment unit 140 (S20). In the present embodiment, a treatment device for performing a treatment by irradiating treatment light is described as an example, but it is also possible to use a treatment device for performing a treatment using other energy sources, such as RF or ultrasound.

When a treatment is performed on a specific treatment location, the storage unit 180 performs a step of storing the treatment location (S30). While the user is moving the handpiece and performing a treatment on a plurality of locations, the treatment locations are cumulatively stored. Furthermore, it is also possible to cumulatively store information on a treatment order for the plurality of treatment locations .

As described above, the step of storing the treatment location may be performed by generating a treatment map and cumulatively recording treatment location information on the map. In this case, the location treated through a first treatment path may be stored in a first treatment map, and a location treated through a second treatment path may be stored in a newly created second treatment map.

Meanwhile, in the aforementioned treatment step (S20), the controller 160 performs the treatment in consideration of a relative location relationship between the location to be treated and previously treated locations . That is, when the location information of the handpiece at the time of a treatment is the same as the previous treatment location stored in the treatment map, at least partially overlaps, or is located within a preset distance from the previous treatment location, a treatment at the corresponding location may be skipped, or a treatment may be performed with a relatively small output to prevent an excessive treatment.

Each of the steps shown in FIG. 8 sequentially illustrates the main steps described above in the skin treatment devices, but the location detecting step may be continuously performed while the treatment is performed on a plurality of different locations, and the treatment step and the treatment location storage step may be repeatedly performed at each location.

FIG. 9 is a flowchart illustrating a control method of a skin treatment device according to the present embodiment in more detail. Hereinafter, the control method of a skin treatment device of the present embodiment will be described in more detail with reference to FIG. 9.

The flowchart of FIG. 9 shows a control operation of the skin treatment device corresponding to one treatment path. In addition, when the user starts a treatment operation, the skin treatment device irradiates treatment light at a preset period, and the user may perform a treatment while continuously moving the location of the handpiece during the treatment. Further, a treatment end time point may be terminated when a preset time arrives from the start of a treatment or when a preset number of treatment lights are irradiated, or may be a time point when there is a user's treatment end operation.

As shown in FIG. 9, prior to performing a treatment, a step of calibrating the skin treatment device 1 may be performed (S 110). In this step, it may be checked whether a direction of irradiation of treatment light is properly aligned in the treatment unit or whether movement information is accurately recognized in the detecting unit, and fine adjustment may be performed. In particular, in the case of the first sensor unit 171, when detecting horizontal movement information in an image manner, recognition errors may occur depending on a skin type and color. Therefore, a calibration step for adjusting this error is performed in advance.

When the skin treatment device is calibrated, a treatment operation starts (S120). This step may be performed in a manner in which the user operates a button to start a treatment. Thereafter, the user proceeds with a treatment on a plurality of locations while moving the location of the handpiece.

First, the handpiece is moved to a first location, which is an initial treatment location, and treatment light is irradiated (S130). Here, the detecting unit 170 detects location information of the first location, and the storage unit generates a treatment map and stores the first location in the treatment map (S 140). Then, the user moves the location of the handpiece again to a second location, which is a next scheduled treatment location (S150). Then, the detecting unit 170 detects the second location information using the first and second sensor units (S160). Thereafter, a step of comparing the detected scheduled treatment location with the treatment map is performed (S170). This step may be a step of determining a relative location relationship between the scheduled treatment location and the previously treated location recorded in the storage unit 180.

Based on a result of the comparison step, the controller 160 determines whether normal treatment conditions are satisfied (S180). These normal treatment conditions may be determined based on the relative location relationship between the scheduled treatment location and the previously treated location, and may be further determined by further considering information related to a treatment timing, such as a treatment order. These normal treatment conditions may be designed variously according to a treatment part, treatment lesion, and treatment intensity.

If it is determined that the normal treatment conditions are satisfied, the controller 160 performs a treatment by irradiating treatment light to the corresponding scheduled treatment location in a first mode corresponding to a normal treatment mode (S190). For example, the treatment is performed in the same manner as the treatment carried out at the first location. Then, by updating the location where the treatment was performed in the treatment map, the corresponding location is stored in the storage unit as a treatment location (S200). Then, after checking whether it is a treatment end point of the corresponding treatment path (S210), if it is not the treatment end point, the handpiece is moved to a next treatment location (a third position) and a treatment is performed up to an N-th location by repeating the steps after S 150, and if it is the end point, the treatment of the corresponding treatment path is terminated (S240).

Meanwhile, if the normal treatment conditions are not satisfied (for example, if the treatment location is the same as the previously treated location, at least partially overlaps, or is located within a preset distance from the previously treated location) according to the comparison result, the controller 160 replaces the treatment for the corresponding treatment location with a second mode control (S220). Here, the second mode may be designed in a variety of control methods, and as an example, it may be controlled in such a manner that a treatment on a corresponding location is skipped or a treatment is performed using relatively less energy. Accordingly, it is possible to prevent overtreatment in a location identical to or adjacent to a previously treated location. Thereafter, after checking whether it is the treatment end point of the corresponding treatment path (S230), if it is not the treatment end point, the handpiece is moved to a next treatment location (a third position) and a treatment is performed up to an N-th location by repeating the steps after S 150, and if it is the end point, the treatment of the corresponding treatment path is terminated (S240).

In the control method of the skin treatment device described above, it is also possible to configure for the user to stop the handpiece at the treatment location, detect the location, and compare the location with the treatment map to control the treatment contents, but according to the control method of the present embodiment, while the user continuously moves the location of the handpiece, each step is quickly performed for each treatment light irradiation period, and the treatment operation considering the previous treatment location is performed.

In addition, since the first location is the first treatment location of the corresponding path, the steps corresponding to S160 to S 180 may not be performed before irradiating treatment light, but the corresponding step is performed at the subsequent treatment locations (second location to N-th position).

Also, as described above, in the present embodiment, the skin treatment device in which treatment light is automatically irradiated at a preset period is used. Accordingly, the step of detecting the scheduled treatment location is performed by detecting the location information at a time when a next irradiation period of treatment light arrives, that is, immediately before treatment light is irradiated in the next irradiation period, while the handpiece is moving. However, if the user's motion is required for every shot, not the treatment light irradiation method according to the present embodiment, in the step of detecting the scheduled treatment location, location information at the time when the user performed a shot operation may be detected as a scheduled treatment location.

## Claims

1. A skin treatment device comprising:
a treatment unit configured to move on a skin surface by a user's motion and perform a treatment by delivering energy to a plurality of treatment locations;
a detecting unit configured to detect location information of the treatment unit while the treatment unit is moving;
a storage unit configured to store information on a treatment location on which treatment has been performed based on information detected by the detecting unit; and
a controller configured to control a treatment operation based on information on the treatment location on which the treatment has been performed.

2. The skin treatment device of claim 1, wherein the storage unit cumulatively stores information on a plurality of treatment locations on which the treatment has been performed.

3. The skin treatment device of claim 1, wherein the storage unit cumulatively stores information on a plurality of treatment locations on which the treatment has been performed and information on a treatment order of each treatment location.

4. The skin treatment device of claim 1, wherein, while the treatment is performed by the treatment unit, the storage unit generates a treatment map recording the treatment location information and cumulatively records the treatment location information on the treatment map.

5. The skin treatment device of claim 4, wherein, while a treatment is performed through a first treatment path, the storage unit records treatment location information corresponding to the first treatment path in a first treatment map, and while a treatment is performed through a second treatment path, the storage unit records treatment location information corresponding to the second treatment path in a second treatment map.

6. The skin treatment device of claim 4, further comprising:
a display unit configured to display the treatment map to a user while the treatment is performed by the treatment unit.

7. The skin treatment device of claim 1, wherein
the detecting unit detects location information of the treatment unit by detecting horizontal movement information according to reference coordinates and rotation information of the reference coordinates, while the treatment unit is moving.

8. The skin treatment device of claim 1, wherein
the detecting unit includes an image sensor configured to acquire movement information by acquiring an image of the skin surface in real time while the treatment unit is moving and a gyro sensor configured to detect rotation information of the treatment unit.

9. The skin treatment device of claim 1, wherein,
when the treatment unit is located in a previously treated treatment location or within a preset distance from the previously treated treatment location, the controller controls the treatment unit not to transmit energy to the skin surface or controls the treatment to be performed with relatively low output energy.

10. The skin treatment device of claim 1, wherein
the treatment unit automatically transmits energy to the skin surface according to a preset mode while moving along the skin surface by a user's motion, and
when the treatment unit is located in a previously treated treatment location or within a preset distance from the previously treated treatment location, the controller controls the treatment unit not to transmit energy to the skin surface or controls the treatment to be performed with relatively low output energy.

11. A control method of a skin treatment device, the control method comprising:
detecting location information of a treatment unit by a detecting unit, while moving on a location of a patient's skin surface by a user's motion;
operating a treatment unit to transmit energy to the skin surface to perform a treatment; and
cumulatively storing information on a treatment location on which a treatment has been performed by the treatment unit based on information detected by the detecting unit,
wherein, in the step of performing the treatment, the controller controls the operation of the treatment unit in consideration of location information of the treatment unit and the information on the treatment location on which the treatment has been performed.

12. The control method of claim 11, wherein
the storing of the treatment location information includes accumulatively storing information on a plurality of treatment locations on which treatment has been performed and information on a treatment order of each treatment location.

13. The control method of claim 11, wherein
the storing of the treatment location information includes generating a treatment map for recording the treatment location information while the treatment is performed by the treatment unit and cumulatively recording the treatment location information on the treatment map.

14. The control method of claim 13, wherein
the storing of the treatment location information includes generating a first treatment map and recording treatment location information by the treatment unit while a treatment is performed through a first treatment path and generating a second treatment map and recording treatment location information while a treatment is performed through a second treatment path.

15. The control method of claim 11, wherein
the detecting of the location information of the treatment unit includes detecting location information of the treatment unit by detecting horizontal movement information according to reference coordinates and rotation information of the reference coordinates while the treatment unit is moving.

16. The control method of claim 11, wherein
when the treatment unit is located in a previously treated treatment location or within a preset distance from the previously treated treatment location, the controller controls the treatment unit not to transmit energy to the skin surface or controls the treatment to be performed with relatively low output energy.

17. A control method of a skin treatment device, the control method comprising:
generating a treatment map for recording information on a treatment location on which a treatment has been performed;
comparing a first location on which a treatment is scheduled with information on a previously treated location recorded in the treatment map;
when the first location satisfies a preset treatment condition according to a comparison result of the comparing operation, transferring energy to the first location to perform a treatment; and
when the treatment is performed on the first location, recording the first location as a previous treatment location on the treatment map.

18. The control method of claim 17, wherein
the preset treatment condition is one of a case in which the first location does not overlap with the previous treatment location recorded on the treatment map, a case in which the first location is spaced apart from the previous treatment location recorded in the treatment map by a preset distance or more, and a case in which the first location has passed a preset time from a previous treatment time.

19. The control method of claim 17, wherein,
when the first location does not satisfy the preset treatment condition as a comparison result of the comparing operation, a treatment is not performed at the first location or a treatment is performed by transmitting a relatively less output.
